# EUROPÄISCHE PATENTSCHRIFT

(11) **EP 2 848 230 B1**
(45) Veröffentlichungstag und Bekanntmachung des Hinweises auf die Patenterteilung: **01.03.2017**
(21) Anmeldenummer: 14179959.3
(22) Anmeldetag: 06.08.2014
(51) Int. Cl.: A61F 2/00, A61B 17/12

(54) **Implantat zur Behandlung einer Hernie**
Implant for treatment of a hernia
Implant destiné au traitement d'une hernie

(30) Priorität: 11.09.2013 DE 102013109959
(43) Veröffentlichungstag der Anmeldung: 18.03.2015
(73) Patentinhaber: FEG Textiltechnik Forschungs- und Entwicklungsgesellschaft mbH, 52070 Aachen (DE)
(72) Erfinder: Löhde, Eckhard, Dr. med., 14129 Berlin (DE)
(74) Vertreter: Gottschald, Jan

(56) Entgegenhaltungen:
- EP-A1- 2 524 670
- WO-A1-02/22047
- WO-A1-2008/060973
- US-A1- 2003 212 462

## Beschreibung

Die Erfindung betrifft ein Implantat zur Behandlung einer Hernie gemäß dem Oberbegriff von Anspruch 1.

Aus dem Stand der Technik sind Implantate bekannt, mit denen Hernien, also Lücken in den tragenden Bauchwandschichten, behandelt werden können. Dabei wird das in der Regel flächige Implantat an dem die Lücke bildenden Durchbruch so positioniert, dass der Durchbruch geschlossen und mittels des Implantats fixiert wird. Es ist gewünscht, dass das Implantat ein Einwachsen des Körpergewebes der umliegenden Bauchwand begünstigt, damit es zu keiner Verschiebung oder gar Ablösung des Implantats von der Bauchwand kommt, was den Heilungsprozess beeinträchtigen würde.

Eine Sondersituation besteht insbesondere bei einer Hiatushernie, also einem Durchbruch an dem Speiseröhrenschlitz. An dem Speiseröhrenschlitz treten die Speiseröhre sowie die beiden Hauptstämme des Nervus vagus aus der Bauchhöhle. Hier ist zwar ein möglichst einfaches Einwachsen des Körpergewebes der Bauchwand selbst, also des Muskelgewebes des Zwerchfells, in das Implantat gewünscht, nicht jedoch eine Verbindung des Implantats mit der Speiseröhre oder des mit ihm verlaufenden Nervus vagus posterior. Eine solche Verbindung ist später nur schwer lösbar.

Aus der EP 2 524 670 A1, welche hier als nächstkommend angesehen wird, ist ein dreidimensionales Implantat mit einem textilen Mesh zur Behandlung von Hernien bekannt, welches einen Mittelsteg und zwei seitliche Flügelstege aufweist, mit denen es im Querschnitt ein T-Profil bildet. Dabei wird der Mittelsteg in den Durchbruch geführt, während die Flügelstege das Körpergewebe um den Durchbruch bedecken. Zur Vermeidung von Verletzungen oder Irritationen, etwa durch an dem Rand abstehende Filamente, ist hier vorgesehen, dass das Implantat an einem freien äußeren Rand umgeschlagen oder mit einer Silikonschicht versehen ist.

Nachteilig an diesem Stand der Technik ist jedoch, dass bei der Behandlung einer Hiatushernie an dem Speiseröhrenschlitz weiterhin das Risiko einer Berührung des Implantats mit der Speiseröhre und dem Nervus vagus posterior und eines resultierenden Einwachsens besteht. Die an dem Rand vorgesehene Silikonschicht verhindert ein Einwachsen nur gemäß ihrer Ausdehnung, wobei das Risiko des Einwachsens der Speiseröhre nicht nur an dem Rand im engeren Sinne, sondern grundsätzlich auch in einem bestimmten Abstand zum Rand besteht. Eine weitere Ausdehnung der aufgetragenen Silikonschicht mit größerem Abstand zum Rand würde die Flexibilität des Implantats einschränken und damit das Einbringen und Befestigen des Implantats beeinträchtigen.

Vor diesem Hintergrund liegt der Erfindung das Problem zugrunde, ein Implantat zur Behandlung einer Hernie bereitzustellen, welche einen Schutz vor einer Verbindung des Implantats mit Körpergewebe, welches nicht zu dem zu verschließenden Durchbruch gehört, bietet und dabei die Flexibilität des Meshs beim Einbringen des Implantats in den Durchbruch bewahrt.

Das obige Problem wird bei einem Implantat gemäß dem Oberbegriff von Anspruch 1 durch die Merkmale des kennzeichnenden Teils von Anspruch 1 gelöst.

Wesentlich für die Erfindung ist die Erkenntnis, dass derjenige Bereich des Implantats, in welchem ein Einwachsen verhindert werden soll, erst nach dem Einbringen des Implantats in dem Durchbruch, vor einem solchen Einwachsen geschützt wird. Während des Einbringens stört eine hierzu vorgesehene und entsprechend angeordnete Adhäsionsbarriere den Einbringvorgang nicht, gewährleistet jedoch nachträglich einen wirksamen Schutz.

Es ist also das Implantat mit einer Adhäsionsbarriere versehen, welche zunächst die Flexibilität des textilen Mesh des Implantats nicht einschränkt und erst nach dem Anbringen dieses Meshs am Durchbruch so platziert wird, dass das Mesh und - im Falle einer Hiatushernie - die Speiseröhre wechselseitig durch eine Abdeckung der relevanten Bereiche vor einer Berührung und einer möglichen Verbindung geschützt sind. Speziell kann damit der zum Speiseröhrenschlitz weisende und diesen abschließende Rand des Mittelstegs eines im Querschnitt T-förmigen Implantats abgedeckt werden.

Die bevorzugten Ausgestaltungen der Ansprüche 2 und 3 betreffen bevorzugte Möglichkeiten, diese Adhäsionsbarriere an dem Implantat zu befestigen.

Der Anspruch 5 stellt bevorzugte Querschnittsausgestaltungen der Steganordnung des Meshs vor.

Vorteile bei der Herstellung und dem Einwachsverhalten in die Bauchwand ergeben sich, wenn der Steg gemäß der Ausgestaltung des Anspruchs 6 durch eine doppelte Lage des Meshs gebildet wird, was etwa durch ein Umschlagen erreicht werden kann. Umgekehrt erhöht dies grundsätzlich das Risiko eines Einwachsens der Speiseröhre, welches Risiko aber wirksam durch die Adhäsionsbarriere vermieden werden kann.

Die bevorzugten Ausgestaltungen der Ansprüche 7 bis 11 wiederum betreffen Möglichkeiten, die Verbindung der Adhäsionsbarriere zum textilen Mesh so auszugestalten, dass die Adhäsionsbarriere während des Einbringens des Implantats an dem Durchbruch diesen Vorgang nicht stört und erst nach Abschluss des Vorgangs zum Abdecken etwa des Stegrandes verformt wird.

Schließlich betrifft die bevorzugte Ausgestaltung des Anspruchs 15 eine Möglichkeit, die Adhäsionsbarriere nach Einbringen des Implantats ohne das Risiko einer Beschädigung der Adhäsionsbarriere befestigen zu können.

Im Folgenden wird die Erfindung anhand einer lediglich Ausführungsbeispiele darstellenden Zeichnung näher erläutert. In der Zeichnung zeigt
- Fig. 1: ein vorschlagsgemäßes Implantat gemäß einem ersten Ausführungsbeispiel mit einer Adhäsionsbarriere in einem Grundzustand,
- Fig. 2: das vorschlagsgemäße Implantat der Fig. 1 mit einer Adhäsionsbarriere in einem Zustand der Verformung nach einem Einbringen des Implantats in einen Durchbruch und
- Fig. 3a-b: jeweils einen Querschnitt von zwei weiteren Ausführungsbeispielen eines vorschlagsgemäßen Implantats.

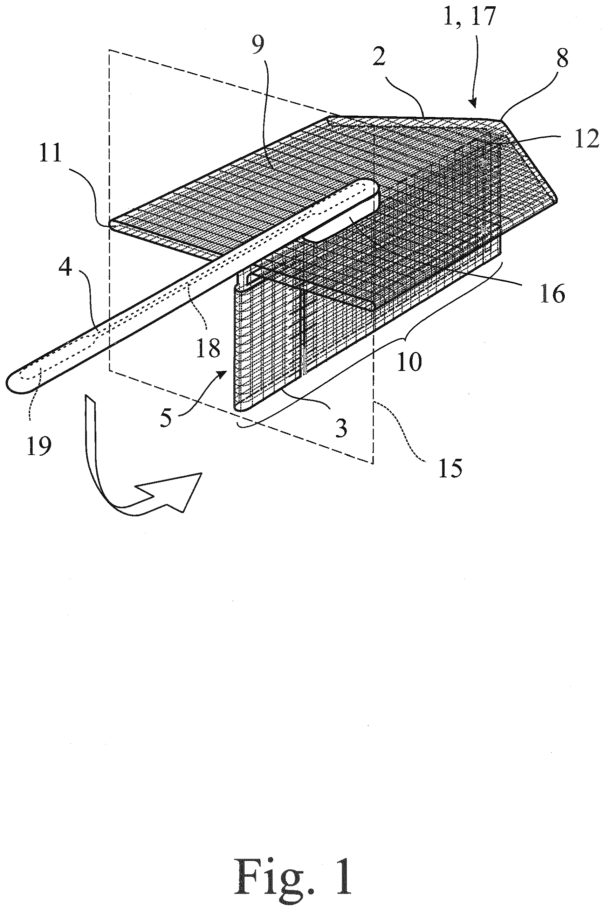

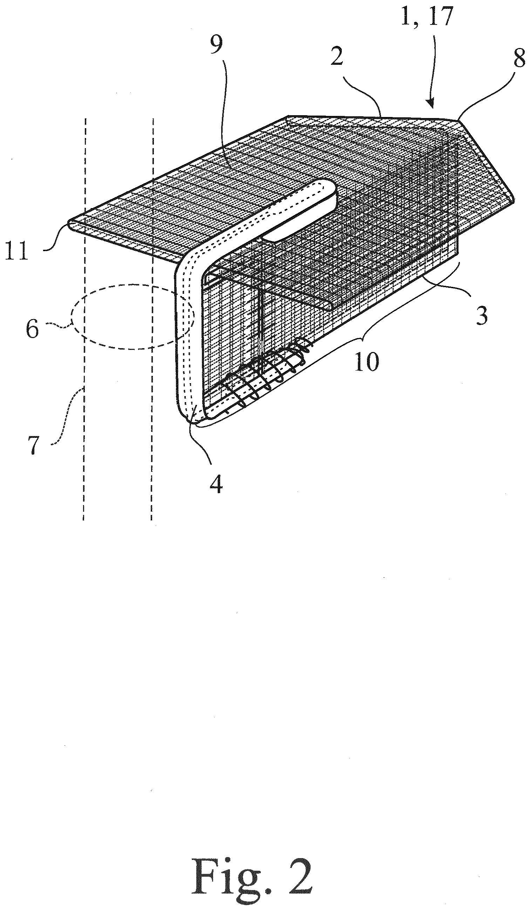

Ein vorschlagsgemäßes Implantat zur Behandlung einer Hernie, wie es in einem ersten Ausführungsbeispiel in den Fig. 1 und 2 dargestellt ist, weist ein textiles Mesh 1 zur Verbindung mit Körpergewebe auf. Bei dem textilen Mesh 1 kann es sich grundsätzlich um ein beliebiges, netzartiges textiles Gebilde handeln, insbesondere um ein Gewebe oder ein Gewirke. Speziell kann es sich um ein in Häkelgalontechnik hergestelltes Kettengewirke handeln. Das textile Mesh 1 kann auch aus mehreren einzelnen solcher textilen Gebilde zusammengesetzt sein. Bevorzugt besteht das textile Mesh 1 zumindest teilweise aus nicht resorbierbarem, biostabilem Polyvinylidenfluorid (PVDF). Verschiedene Zusatzstoffe, etwa zur Sichtbarmachung in bestimmten Untersuchungsverfahren, oder sonstige Strukturen können ebenfalls in oder an dem textilen Mesh 1 vorgesehen sein.

Dieses Mesh 1 weist eine Grundfläche 2 und eine von der Grundfläche 2 abragende Steganordnung 3 auf. Sowohl die Grundfläche 2 als auch die Steganordnung 3 sind also von dem textilen Mesh 1 umfasst, können aber auch erst nachträglich zu dem textilen Mesh 1 zusammengefügt worden sein, brauchen also kein durchgehendes einzelnes Gewirke, Gewebe oder eine sonstige einheitliche textile Struktur zu bilden. Das textile Mesh 1 kann neben der abragenden Steganordnung 3 noch weitere textile oder nicht textile Strukturen aufweisen, welche in grundsätzlich beliebiger Form mit der Grundfläche 2 oder der Steganordnung 3 verbunden sein können.

Das vorschlagsgemäße Implantat kann, wie in dem in den Fig. 1 und 2 gezeigten ersten Ausführungsbeispiel, zur Behandlung einer Hiatushernie vorgesehen sein. Das vorschlagsgemäße Implantat kann ebenso zur Behandlung einer Inguinalhernie vorgesehen sein. Nachfolgend wird die Funktionsweise des vorschlagsgemäßen Implantats jedoch lediglich am Beispiel der Behandlung einer Hiatushernie beschrieben.

Gekennzeichnet ist das vorschlagsgemäße Implantat dadurch, dass das Implantat eine folienartige, vorzugsweise längliche, Adhäsionsbarriere 4 aufweist, welche zur Abdeckung eines Stegrands 5 der Steganordnung 3 ausgerichtet und/oder positioniert ist. Die Adhäsionsbarriere 4 ist folienartig in dem Sinne, dass sie keine Maschen, Poren oder sonstigen Öffnungen oder Durchgänge aufweist, in welche etwa Körpergewebe einwachsen konnte. Vorzugsweise besteht sie aus hydrophobem Material und steht einer Verbindung mit Körpergewebe, insbesondere einer Verbindung durch Einwachsen, entgegen.

Die Adhäsionsbarriere 4 ist an dem Implantat so ausgerichtet und/oder positioniert, dass sie zur Abdeckung des Stegrands 5, welcher grundsätzlich ein beliebiger Rand der Steganordnung 3 sein kann, geeignet ist. Insbesondere kann die Adhäsionsbarriere 4 zu diesem Zwecke verformbar oder sonst wie in ihrer geometrischen Konfiguration veränderbar sein, um den Stegrand 5 abdecken und auf diese Weise eine Verbindung mit bzw. ein Einwachsen von Körpergewebe an dieser Stelle der Steganordnung 3 verhindern zu können.

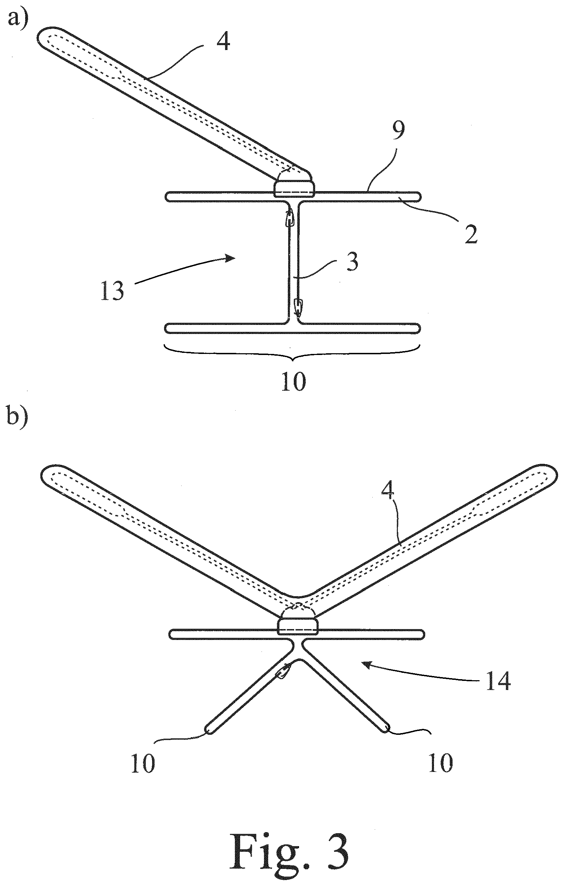

Die Ausrichtung der Adhäsionsbarriere 4 zur Abdeckung des Stegrands 5 kann etwa umfassen, dass die Adhäsionsbarriere 4 - wie in den Fig. 1 und 3 dargestellt - eine längliche Form aufweist und in eine Flächenrichtung bezogen auf die Grundfläche 2 so ausgerichtet ist, dass ein Biegen oder Klappen der so ausgerichteten Adhäsionsbarriere 4 um einen Rand der Grundfläche 2 zu der Abdeckung des Stegrands 5 führt. Insbesondere kann die Adhäsionsbarriere 4 auch so ausgerichtet sein, dass der Stegrand 5 an die Grundfläche 2 in einem Bereich angrenzt, in welchem die Adhäsionsbarriere 4 gemäß ihrer Ausrichtung auf den Rand der Grundfläche 2 trifft. Ferner kann die Adhäsionsbarriere 4 so ausgerichtet sein, dass die Adhäsionsbarriere 4 zu einer verwindungsfreien Abdeckung des Stegrands 5 eingerichtet ist, also für die Abdeckung des Stegrands 5 nicht um eine eigene Längsachse gewunden oder verzwirbelt wird.

Neben den soben beschriebenen Aspekten kann die Positionierung der Adhäsionsbarriere 4, insbesondere die Position einer Befestigung der Adhäsionsbarriere 4 mit dem Implantat, zur Abdeckung des Stegrands 5 zunächst umfassen, dass der Abstand zwischen Adhäsionsbarriere 4 und Stegrand 5 bzw. zwischen der Befestigung der Adhäsionsbarriere 4 und dem Stegrand 5 die Abdeckung des Stegrands 5 erlaubt. Für den Abstand in diesem Sinne ist der wirksame, also durch die Adhäsionsbarriere 4 zu überwindende Abstand maßgeblich.

In der Fig. 2, welche sich ja auf den eingebrachten Zustand des Implantats bezieht, ist zur Verdeutlichung der Schutzfunktion der Adhäsionsbarriere 4 schematisch die Lage des Speiseröhrenschlitzes 6 und der Verlauf der Speiseröhre 7 wiedergegeben.

Grundsätzlich ist die Steganordnung 3 als Bestandteil des textilen Meshs 1 ja für eine solche Verbindung mit Körpergewebe im Bereich des zu behandelnden Durchbruchs vorgesehen. Indem nun also die Adhäsionsbarriere 4 durch diese nachträgliche Abdeckung den Stegrand 5 der Steganordnung 3 vor einer Verbindung - hier speziell mit der Speiseröhre 7 und dem Nervus vagus posterior - schützt, brauchen keine sonstigen Maßnahmen zur Verhinderung dieses Einwachsens am Stegrand 5 vorgesehen zu sein, welche unter Umständen die Biegsamkeit oder sonstige Gestaltbarkeit des textilen Meshs 1 an diesem Teil der Steganordnung 3 einschränken könnten.

Bevorzugt wird bei einem textilen Mesh 1 mit einer Grundfläche 2 und einer von der Grundfläche abragenden Steganordnung 3 das Implantat so eingebracht, dass die Grundfläche 2 auf der Innenseite des Durchbruchs und damit in der Bauchhöhle angeordnet ist. Entsprechend weist bzw. ragt die Steganordnung 3 nach außen - bezogen auf die Bauchhöhle - durch den Durchbruch. Die in den Fig. 1 und 2 erkennbare Pfeilung 8 der Grundfläche 2 am dem Stegrand 5 gegenüberliegenden Ende des Implantats erleichtert diesen Vorgang. In diesem Zusammenhang ist bevorzugt vorgesehen, dass die Adhäsionsbarriere 4 an der Grundfläche 2 befestigt ist. Es entfällt die Notwendigkeit, eine Befestigung der Adhäsionsbarriere 4 an dem textilen Mesh 1 nach dem Einbringen des Implantats innerhalb der Bauchhöhle vornehmen zu müssen.

Um das Einwachsen des Körpergewebes in die Grundfläche 2 durch die Adhäsionsbarriere 4 nicht zu beeinträchtigen, ist bevorzugt vorgesehen, dass die Adhäsionsbarriere 4 auf einer der Steganordnung 3 abgewandten Rückseite 9 der Grundfläche 2 befestigt ist. Diese Rückseite 9 entspricht also derjenigen Seite der Grundfläche 2, welcher in Richtung der Innenseite des Durchbruchs und damit in das Innere Bauchhöhle weist.

Hiermit wäre also ein erster bevorzugter Befestigungsort der Adhäsionsbarriere 4 an dem Implantat beschrieben. Wie auch aus dem Ausführungsbeispiel der Fig. 1 und 2 hervorgeht, ist es weiterhin vorteilhaft, dass die Adhäsionsbarriere 4 zur insbesondere weiteren Befestigung an einem Vorderbereich 10 der Steganordnung 3 eingerichtet ist, welcher Vorderbereich 10 zu der Grundfläche 2 beabstandet angeordnet ist. Die Steganordnung 3 trennt also räumlich, durch ihre Ausdehnung weg von der Grundfläche 2, diesen Vorderbereich 10 von eben dieser Grundfläche 2. Dabei kann es sich, wie in den Fig. 1 und 2, dargestellt bei dem Vorderbereich 10 um einen Bereich und damit einen Teil der Steganordnung 3 selbst handeln, also einen Bereich insbesondere des textilen Meshs 1 der Steganordnung 3. Es kann aber auch ebenso eine beliebige andere, textile oder nicht-textile Struktur an der Steganordnung 3 angeordnet sein, welche dann ebenfalls zu der Grundfläche 2 beabstandet wäre und einen Vorderbereich 10 im obigen Sinne bilden würde. Bei der Behandlung einer Hiatushernie muss die Adhäsionsbarriere 4 nicht den zu behandelnden Durchbruch selbst passieren, sondern kann durch den Speiseröhrenschlitz 6 von der Rückseite 9 der Grundfläche 2 zu dem genannten Vorderbereich 10 geführt werden. Auf diese Weise stört sie den Heilungsprozess im Durchbruch selbst nicht und kann gleichzeitig den Verbindungsschutz zwischen Speiseröhre 7 und dem gegenüber der Speiseröhre 7 exponierten Stegrand 5 bereitstellen.

Wie ebenfalls in dem ersten Ausführungsbeispiel der Fig. 1 und 2 dargestellt, ist es bevorzugt, dass die Grundfläche 2 einen Querrand 11 aufweist und die Steganordnung 3 entlang einer im Wesentlichen senkrecht zum Querrand 11 verlaufenden Längsstrecke 12 an der Grundfläche 2 befestigt ist. Insbesondere kann hier die Adhäsionsbarriere 4 ebenfalls in einem Abschnitt der Längsstrecke 12 - auch an der der Steganordnung 3 abgewandten Rückseite 9 der Grundfläche 2 - an der Grundfläche 2 befestigt sein. Ebenso ist es vorteilhafterweise bevorzugt, dass die Adhäsionsbarriere im Wesentlichen parallel zu der Längsstrecke 12 ausgerichtet sein.

Diese Anordnung kommt der Behandlung am Speiseröhrenschlitz 6 entgegen, wo also die Steganordnung 3 mit der Längsstrecke 9 entlang und innerhalb des Durchbruchs angeordnet wird. Damit bildet der Querrand 11 zumindest abschnittsweise die Abgrenzung zum Speiseröhrenschlitz 6, in welchem Bereich sie potenziell mit der Speiseröhre 7 in Berührung kommen könnte. Dieser Bereich ist dann bevorzugt gegenüber einer Verbindung mit der Speiseröhre 7 zu schützen, was wie beschrieben durch die entsprechende Anordnung der Adhäsionsbarriere 4 erfolgt. Die Breite der Adhäsionsbarriere 4 kann dabei so gewählt werden, dass derjenige Bereich des Querrands 11, welcher vor einer Verbindung geschützt werden soll, durch die Adhäsionsbarriere 4 abgedeckt wird.

Gemäß dem Ausführungsbeispiel der Fig. 1 und 2 ist es bevorzugt, dass die Grundfläche 2 und die Steganordnung 3 entlang der Längsstrecke 12 im Querschnitt als T-Profil ausgebildet sind. Die Steganordnung 3 wird in diesem Fall also erkennbar über eine einzelne, im Wesentlichen rechteckförmige und im Wesentlichen senkrecht zur Grundfläche ausrichtbare Fläche gebildet.

Möglich ist es aber auch, dass die Grundfläche 2 und die Steganordnung 3 entlang der Längsstrecke 9 im Querschnitt als H-Profil 13, wie in der Figur 3a dargestellt, oder als K-Profil 14, wie in der Fig. 3b dargestellt, ausgebildet sind. Die Ausgestaltung als H-Profil 13 ermöglicht eine beidseitige, großflächige Kontaktierung des Körpergewebes in der Umgebung des Durchbruchs. Die Ausgestaltung als K-Profil 14 bietet einen ähnlichen Vorteil, wenn die Dicke des zu behandelnden Durchbruchs, also etwa die Dicke des Zwerchfells, geringer ist. In diesem Fall kann insbesondere eine Adhäsionsbarriere 4 mit zwei Armen vorgesehen sein. Es kommen auch weitere Querschnittsausgestaltungen mit entsprechend unterschiedlich vielen Armen der Adhäsionsbarriere 4 infrage.

Es kann das textile Mesh 1 mit seiner Grundfläche 2 und der Steganordnung 3 durch eine Faltung eines flächigen Grundmusters geformt sein. Dabei kommt es dazu, dass, wie bevorzugt, die Steganordnung 3 und insbesondere der Stegrand 5 eine Mehrfachlage des Meshs 1 aufweist. Dies ist auf der einen Seite vorteilhaft, weil damit ein Einwachsen des Körpergewebes des Durchbruchs entlang der Länge der Steganordnung 3 begünstigt wird, auf der anderen Seite aber steigt entsprechend auch das Verbindungsrisiko am Stegrand 5 mit der Speiseröhre 7. Diesem Risiko kann jedoch wirksam mit der vorschlagsgemäßen Adhäsionsbarriere 4 begegnet werden, sodass lediglich die Vorteile einer solchen Ausgestaltung verbleiben.

Bevorzugt ist ferner, dass der Stegrand 5 in dieselbe Richtung wie der Querrand 11 weist und dass hier insbesondere der Stegrand 5 im Wesentlichen in einer Randebene 15 mit dem Querrand 11 verläuft. Die Anordnung des Stegrands 5 in einer Randebene 15 mit dem Querrand 11 erleichtert ein gemeinsames Abdecken sowohl des Stegrands 5 als auch des Querrands 11 in den relevanten Bereichen durch die Adhäsionsbarriere 4. Vorzugsweise ist die Adhäsionsbarriere 4 so ausgerichtet, dass die Adhäsionsbarriere 4 auf einen Schnittbereich, also den Bereich eines Zusammentreffens, zwischen Stegrand 5 und Querrand 11 weist.

Wie aus einem Vergleich der Fig. 1 und der Fig. 2 des ersten Ausführungsbeispiels erkenntlich ist, ist die Adhäsionsbarriere 4 vorzugsweise dazu eingerichtet, zur Abdeckung des Stegrands 5 und zur zumindest teilweisen Abdeckung des Querrands 11 gebogen und befestigt zu werden. Vor diesem Biegen und Befestigen kann also die Adhäsionsbarriere 4 so angeordnet werden, dass sie dem Einbringen und Befestigen des Implantats und hier speziell der Grundfläche 2 und der Steganordnung 3 des textilen Meshs 1 nicht erschwert. Die Befestigung der Adhäsionsbarriere 4 an der Steganordnung 3 bzw. an dem Vorderbereich 10 der Steganordnung 3 kann auf grundsätzlich beliebige geeignete Art und Weise erfolgen. Denkbar wäre hier sowohl ein Vernähen als auch ein Tackern, Kleben oder eine Temperaturbehandlung.

Wie bereits bemerkt, soll das Vorsehen der Adhäsionsbarriere 4 als Teil des Implantats die mechanischen Eigenschaften des textilen Mesh 1 und insbesondere seine Verformbarkeit nach Möglichkeit nicht beeinflussen. Hierzu ist bevorzugt vorgesehen, dass das Implantat einen, vorzugsweise stabilen, Stützsockel 16 umfasst, über welchen die Adhäsionsbarriere 4 an dem Mesh 1 und hier vorzugsweise an der Grundfläche 2 befestigt ist, und welcher Stützsockel 16 bei einer Verformung des Meshs 1 im Wesentlichen starr bleibt.

Damit dieser Stützsockel 16 die Verformbarkeit der Grundfläche 2 in demjenigen Bereich, in welchem die Grundfläche sich ggf. dem Verlauf des Speiseröhrenschlitzes 6 anpassen muss, nicht einschränkt, ist dieser Stützsockel 16 vorzugsweise beabstandet zu dem Querrand 11 angeordnet. Der Abstand des Stützsockels 16 zum Querrand 11 kann dabei zwischen 4 und 6 mm und bevorzugt 5 mm betragen. Es kann auf diese Weise eine stabile Befestigung der Adhäsionsbarriere 4 an der Grundfläche 2 erreicht werden, welche aber weitgehend eine Verformbarkeit des Meshs 1 erhält, so als ob keine Adhäsionsbarriere 4 vorhanden wäre.

Zur näheren Ausgestaltung des Stützsockels 16 ist bevorzugt vorgesehen, dass dieser länglich geformt ist und im Wesentlichen entlang zumindest eines Teils der Längsstrecke 12 befestigt ist, und zwar vorzugsweise an der Rückseite 9 der Grundfläche 2. Es handelt sich hier um einen verhältnismäßig verformungsfreien Bereich des Implantats. Die längliche Form des Stützsockels 16 kann in Richtung der Längsstrecke 12 und/oder gemäß einer Längsrichtung des Adhäsionsbarriere 4 ausgerichtet sein.

Die Flexibilität des textilen Meshs 1 wird weiter dadurch gewährleistet, dass wie bevorzugt und in den Fig. 1, 2 sowie 3a und 3b dargestellt, der Stützsockel 16 eine Sockelhöhe aufweist, welche einen Sockelabstand von zwischen 0,5 und 1,5 Millimetern und vorzugsweise von einem Millimeter zwischen der Adhäsionsbarriere 4 und der Grundfläche 2 herstellt. Ein solcher Stützsockel 16 kann insbesondere dadurch einfach hergestellt und mit der Adhäsionsbarriere 4 verbunden werden, dass der Stützsockel 16 einstückig mit der Adhäsionsbarnere 14 ausgestaltet ist. Es handelt sich also bevorzugt um eine zusammenhängende Struktur aus demselben Material.

Umgekehrt ist es ebenso bevorzugt, dass die Grundfläche 2 und die Steganordnung 3 von einem einzelnen Meshstück 17 und hier vorzugsweise von einem einzelnen Gewirke umfasst sind. Damit bilden die Grundfläche 2 und die Steganordnung 3 ein einzelnes zusammenhängendes Netz und vorzugsweise ein einstückiges Gewirke, Gewebe oder sonstiges textiles Gebilde, sodass sie in einem einzelnen Arbeitsgang hergestellt werden und dann etwa durch eine Faltung in die gewünschte Form gebracht werden können. Entsprechend ist bevorzugt vorgesehen, dass die Steganordnung 3 durch eine Faltung des Meshstücks 17 gebildet ist. Vorzugsweise wird die Faltung dabei so vorgenommen, dass gemäß der Darstellung der Fig. 1 und 2 der Stegrand 5 durch eine umgeschlagene Kante des Meshstücks 17 gebildet wird.

Als bevorzugtes Material für die Adhäsionsbarriere 4 kommt Silikon in Betracht, welches zur Verhinderung einer Verbindung und gleichzeitig hinsichtlich seiner Biokompatibilität vorteilhafte Eigenschaften aufweist. Daher ist bevorzugt, dass die Adhäsionsbarriere 4 zumindest teilweise und vorzugsweise vollständig aus Silikon besteht.

Es erleichtert das Einbringen des Implantats in den Durchbruch und die anschließende Befestigung, wenn die Adhäsionsbarriere 4 während dieses Vorgangs gleichsam selbstständig von dem Durchbruch ferngehalten wird. Das kann dadurch erreicht werden, dass vorzugsweise die Adhäsionsbarriere 4 eine Verstärkung 18 zum Strecken der Adhäsionsbarriere 4 im entspannten Zustand aufweist, wobei die gestreckte Adhäsionsbarriere 4 im Wesentlichen parallel zu der Grundfläche 2, vorzugsweise entlang der Längsstrecke 12, verläuft. Dieser Zustand ist speziell in der Fig. 1 dargestellt. Die Verstärkung 18 kann eine Versteifung sein, welche etwa durch eine eingebrachte Strebe oder durch eine Verdickung des Materials der Adhäsionsbarriere 4, z. B. des Silikons, gebildet sein kann. Sie tendiert also im entspannten Zustand zu einer Streckung der Adhäsionsbarriere 4, wodurch während des Einbringens des Implantats die Adhäsionsbarriere 4 erst einmal von dem Durchbruch und den Befestigungsstellen des Meshs 1 ferngehalten wird.

Nach dem erfolgten Einbringen und der Befestigung des Meshs 1 kann dann die Adhäsionsbarriere 4 etwa mittels eine Greifzange erfasst und so gebogen werden, dass sie mit der Steganordnung 3 bzw. mit dem obigen Vorderbereich 10 der Steganordnung 3 verbunden werden kann, wie in der Fig. 2 dargestellt ist.

Damit bei dieser Verbindung zwischen Adhäsionsbarriere 4 und der Steganordnung 3 bzw. dem Vorderbereich 10 die Adhäsionsbarriere 4 nicht beschädigt oder gar durchtrennt wird, ist bevorzugt vorgesehen, dass die Adhäsionsbarriere eine örtliche biegsame Schutzverstärkung 19 zur Befestigung an der Grundfläche 2 oder - gemäß dem Ausführungsbeispiel der Fig. 1 und 2 - am Vorderbereich 10 aufweist. Diese Schutzverstärkung 19 ist bevorzugt in ein Material der Adhäsionsbarriere 4, so also etwa in das Silikon, eingearbeitet. Die Schutzverstärkung 19 kann eine Breite von im Wesentlichen 2 mm und zusätzlich oder alternativ eine Dicke zwischen 1 mm und 2 mm aufweisen.

## Patentansprüche

1. Implantat zur Behandlung einer Hernie, vorzugsweise einer Hiatushernie, mit einem textilen Mesh (1) zur Verbindung mit Körpergewebe, welches Mesh (1) eine Grundfläche (2) und eine von der Grundfläche (2) abragende Steganordnung (3) aufweist,
wobei das Implantat eine folienartige Adhäsionsbarriere (4) aufweist, welche in dem Sinne folienartig ist, dass sie keine Maschen, Poren oder sonstigen Öffnungen oder Durchgänge aufweist, in welche etwa Körpergewebe einwachsen könnte, und welche zur Abdeckung eines Stegrands (5) der Steganordnung (3) ausgerichtet und/oder positioniert ist
**dadurch gekennzeichnet,**
**dass** die Adhäsionsbarriere (4) verformbar oder sonst wie in ihrer geometrischen Konfiguration veränderbar ist, um den Stegrand (5) abdecken und auf diese Weise eine Verbindung mit bzw. ein Einwachsen von Körpergewebe an dieser Stelle der Steganordnung (3) verhindern zu können.

2. Implantat nach Anspruch 1, **dadurch gekennzeichnet, dass** die Adhäsionsbarriere (4) an der Grundfläche (2), vorzugsweise auf einer der Steganordnung (3) abgewandten Rückseite (9) der Grundfläche (2), befestigt ist.

3. Implantat nach Anspruch 1 oder 2, **dadurch gekennzeichnet, dass** die Adhäsionsbarriere (4) zur Befestigung an einem Vorderbereich (10) der Steganordnung (3) eingerichtet ist, welcher Vorderbereich (10) zu der Grundfläche (2) beabstandet angeordnet ist.

4. Implantat nach einem der Ansprüche 1 bis 3, **dadurch gekennzeichnet, dass** die Grundfläche (2) einen Querrand (11) aufweist und die Steganordnung (3) entlang einer im Wesentlichen senkrecht zum Querrand (11) verlaufenden Längsstrecke (12) an der Grundfläche (2) befestigt ist.

5. Implantat nach Anspruch 4, **dadurch gekennzeichnet, dass** die Grundfläche (2) und die Steganordnung (3) entlang der Längsstrecke (12) im Querschnitt als T-Profil oder als H-Profil (13) oder als K-Profil (14) ausgebildet sind.

6. Implantat nach Anspruch 4 oder 5, **dadurch gekennzeichnet, dass** der Stegrand (5) eine Mehrfachlage des Meshs (1) aufweist, vorzugsweise, dass der Stegrand (5) in dieselbe Richtung wie der Querrand (11) weist, insbesondere, wobei der Stegrand (5) im Wesentlichen in einer Randebene (15) mit dem Querrand (11) verläuft.

7. Implantat nach Anspruch 6, **dadurch gekennzeichnet, dass** die Adhäsionsbarriere (4) dazu eingerichtet ist, zur Abdeckung des Stegrands (5) und zur zumindest teilweisen Abdeckung des Querrands (11) gebogen und befestigt zu werden.

8. Implantat nach einem der Ansprüche 4 bis 7, **dadurch gekennzeichnet, dass** das Implantat einen, vorzugsweise stabilen, Stützsockel (16) umfasst, über welchen die Adhäsionsbarriere (4) an dem Mesh (1), vorzugsweise an der Grundfläche (2), befestigt ist und welcher Stützsockel (16) bei einer Verformung des Mesh (1) im Wesentlichen starr bleibt, insbesondere, wobei der Stützsockel (16) beabstandet zu dem Querrand (11) angeordnet ist.

9. Implantat nach Anspruch 8, **dadurch gekennzeichnet, dass** der Stützsockel (16) länglich geformt ist und im Wesentlichen entlang zumindest eines Teils der Längsstrecke (12), vorzugsweise, an der Rückseite (9) der Grundfläche (2), befestigt ist.

10. Implantat nach Anspruch 8 oder 9, **dadurch gekennzeichnet, dass** der Stützsockel (16) eine Sockelhöhe aufweist, welche einen Sockelabstand von zwischen 0,5 und 1,5 Millimetern, vorzugsweise von einem Millimeter, zwischen der Adhäsionsbarriere (4) und der Grundfläche (2) herstellt.

11. Implantat nach einem der Ansprüche 8 bis 10, **dadurch gekennzeichnet, dass** der Stützsockel (16) einstückig mit der Adhäsionsbarriere (4) ausgestaltet ist.

12. Implantat nach einem der Ansprüche 1 bis 11, **dadurch gekennzeichnet, dass** die Grundfläche (2) und die Steganordnung (3) von einem einzelnen Meshstück (17), vorzugsweise von einem einzelnen Gewirke, umfasst sind, insbesondere, wobei die Steganordnung (3) durch eine Faltung des Meshstücks (17) gebildet ist.

13. Implantat nach einem der Ansprüche 1 bis 12, **dadurch gekennzeichnet, dass** die Adhäsionsbarriere (4) zumindest teilweise, vorzugsweise vollständig, aus Silikon besteht.

14. Implantat nach einem der Ansprüche 1 bis 13, **dadurch gekennzeichnet, dass** die Adhäsionsbarriere (4) eine Verstärkung (18) zum Strecken der Adhäsionsbarriere (4) im entspannten Zustand aufweist, wobei die gestreckte Adhäsionsbarriere (4) im Wesentlichen parallel zu der Grundfläche (2) verläuft.

15. Implantat nach einem der Ansprüche 1 bis 14, **dadurch gekennzeichnet, dass** die Adhäsionsbarriere (4) eine örtliche biegsame Schutzverstärkung (19) zur Befestigung an der Grundfläche (2) und/oder am Vorderbereich (10) aufweist, vorzugsweise, welche Schutzverstärkung (19) in ein Material der Adhäsionsbarriere (4) eingearbeitet ist.

## Claims

1. Implant for treatment of a hernia, preferably a hiatus hernia, with a textile mesh (1) for connection to body tissue, which mesh (1) has a main surface (2) and, protruding from the main surface (2), a web arrangement (3),
wherein the implant has a film-like adhesion barrier (4), which is film-like in the sense that it has no meshes, pores or other openings or passages into which body tissue, for example, could grow, and which is designed and/or positioned to cover a web edge (5) of the web arrangement (3),
**characterized in that**
the adhesion barrier (4) is deformable or otherwise modifiable in terms of its geometric configuration so as to be able to cover the web edge (5) and in this way prevent a connection to body tissue or an inward growth of body tissue at this location of the web arrangement (3).

2. Implant according to Claim 1, **characterized in that** the adhesion barrier (4) is secured on the main surface (2), preferably on a rear face (9) of the main surface (2) directed away from the web arrangement (3).

3. Implant according to Claim 1 or 2, **characterized**
**in that** the adhesion barrier (4) is designed for securing on a front area (10) of the web arrangement (3), which front area (10) is arranged at a distance from the main surface (2).

4. Implant according to one of Claims 1 to 3, **characterized in that** the main surface (2) has a transverse edge (11), and the web arrangement (3) is secured on the main surface (2) along a longitudinal extent (12) running substantially perpendicular to the transverse edge (11).

5. Implant according to Claim 4, **characterized in that** the main surface (2) and the web arrangement (3) along the longitudinal extent (12) are designed in cross section as a T-shaped profile or as an H-shaped profile (13) or as a K-shaped profile (14).

6. Implant according to Claim 4 or 5, **characterized in that** the web edge (5) has a multiple layer of the mesh (1), preferably **in that** the web edge (5) faces in the same direction as the transverse edge (11), in particular wherein the web edge (5) runs substantially in an edge plane (15) with the transverse edge (11).

7. Implant according to Claim 6, **characterized in that** the adhesion barrier (4) is designed to be bent and secured so as to cover the web edge (5) and to at least partially cover the transverse edge (11).

8. Implant according to one or Claims 4 to 7, **characterized in that** the implant comprises a preferably stable support base (16) via which the adhesion barrier (4) is secured on the mesh (1), preferably on the main surface (2), and which support base (16) remains substantially rigid upon a deformation of the mesh (1), in particular wherein the support base (16) is arranged at a distance from the transverse edge (11).

9. Implant according to Claim 8, characterise in that the support base (16) has an elongate shape and is secured substantially along at least part of the longitudinal extent (12), preferably on the rear face (9) of the main surface (2).

10. Implant according to Claim 8 or 9, **characterized in that** the support base (16) has a base height which produces a base distance of between 0.5 and 1.5 millimetres, preferably of one millimetre, between the adhesion barrier (4) and the main surface (2).

11. Implant according to one of Claims 8 to 10, **characterized in that** the support base (16) is designed in one piece with the adhesion barrier (4).

12. Implant according to one of Claims 1 to 11, **characterized in that** the main surface (2) and the web arrangement (3) are comprised by an individual mesh piece (17), preferably by an individual knit, in particular wherein the web arrangement (3) is formed by a folding of the mesh piece (17).

13. Implant according to one of Claims 1 to 12, **characterized in that** the adhesion barrier (4) is made at least partially, preferably completely, of silicone.

14. Implant according to one of Claims 1 to 13, **characterised in that** the adhesion barrier (4) has a reinforcement (18) for stretching the adhesion barrier (4) in the untensioned state, wherein the stretched adhesion barrier (4) runs substantially parallel to the main surface (2).

15. Implant according to one of Claims 1 to 14, **characterized in that** the adhesion barrier (4) has a local, flexible protective reinforcement (19) for securing on the main surface (2) and/or on the front area (10), which protective reinforcement (19) is preferably worked into a material of the adhesion barrier (4).

## Revendications

1. Implant destiné au traitement d'une hernie, de préférence d'une hernie diaphragmatique, avec un treillis textile (1) à relier au tissu corporel, treillis (1) qui présente une face de base (2) et un agencement de nervure (3) saillant à partir de la face de base (2),
dans lequel l'implant présente une barrière d'adhérence en forme de feuille (4), qui est en forme de feuille en ce sens qu'elle ne présente pas de mailles, de pores ou d'autres ouvertures ou passages, dans lesquels/- lesquelles par exemple un tissu corporel pourrait croître, et qui est orientée et/ou positionnée de façon à recouvrir un bord de nervure (5) de l'agencement de nervure (3),
**caractérisé en ce que** la barrière d'adhérence (4) est déformable ou autrement modifiable comme au niveau de sa configuration géométrique, afin de recouvrir le bord de nervure (5) et de cette façon de pouvoir empêcher une liaison ou une croissance de tissu corporel à cet endroit de l'agencement de nervure (3).

2. Implant selon la revendication 1, **caractérisé en ce que** la barrière d'adhérence (4) est fixée à la face de base (2), de préférence sur un côté arrière (9) de la face de base (2) situé à l'opposé de l'agencement de nervure (3).

3. Implant selon la revendication 1 ou 2, **caractérisé en ce que** la barrière d'adhérence (4) est conçue pour la fixation à une région antérieure (10) de l'agencement de nervure (3), région antérieure (10) qui est disposée à distance de la face de base (2).

4. Implant selon l'une quelconque des revendications 1 à 3, **caractérisé en ce que** la face de base (2) présente un bord transversal (11) et l'agencement de nervure (3) est fixé à la face de base (2) le long d'une zone longitudinal (12) qui est essentiellement perpendiculaire au bord transversal (11).

5. Implant selon la revendication 4, **caractérisé en ce que** la face de base (2) et l'agencement de nervure (3) sont configurés le long de la zone longitudinal (12) avec une section transversale en forme de profilé en T ou de profilé en H (13) ou de profilé en K (14).

6. Implant selon la revendication 4 ou 5, **caractérisé en ce que** le bord de nervure (5) présente une couche multiple du treillis (1), de préférence **en ce que** le bord de nervure (5) est orienté dans la même dilection que le bord transversal (11), en particulier dans lequel le bord de nervure (5) s'étend essentiellement dans un plan de bord (15) avec le bord transversal (11).

7. Implant selon la revendication 6, **caractérisé en ce que** la barrière d'adhérence (4) est conçue pour être pliée et fixée pour le recouvrement du bord de nervure (5) et pour le recouvrement au moins partiel du bord transversal (11).

8. Implant selon l'une quelconque des revendications 4 à 7, **caractérisé en ce que** l'implant comprend un socle d'appui (16), de préférence stable, par lequel la barrière d'adhérence (4) est fixée au treillis (1), de préférence à la face de base (2), et socle d'appui (16) qui reste essentiellement rigide lors d'une déformation du treillis (1), en particulier dans lequel le socle d'appui (16) est disposé à distance du bord transversal (11).

9. Implant selon la revendication 8, **caractérisé en ce que** le socle d'appui (16) est de forme allongée et est fixé essentiellement le long d'au moins une partie de la zone longitudinale (12), de préférence sur le côté arrière (9) de la face de base (2).

10. Implant selon la revendication 8 ou 9, **caractérisé en ce que** le socle d'appui (16) présente une hauteur de socle, qui établit une distance de socle comprise entre 0,5 et 1,5 millimètres, de préférence d'un millimètre, entre la barrière d'adhérence (4) et la face de base (2).

11. Implant selon l'une quelconque des revendications 8 à 10, **caractérisé en ce que** le socle d'appui (16) est réalisé d'une seule pièce avec la barrière d'adhérence (4).

12. Implant selon l'une quelconque des revendications 1 à 11, **caractérisé en ce que** la face de base (2) et l'agencement de nervure (3) sont entourés d'une seule pièce de treillis (17), de préférence d'une seule pièce de tricot, en particulier dans lequel l'agencement de nervure (3) est formé par un pliage de la pièce de treillis (17).

13. Implant selon l'une quelconque des revendications 1 à 12, **caractérisé en ce que** la barrière d'adhérence (4) est constituée au moins partiellement, de préférence entièrement, de silicone.

14. Implant selon l'une quelconque des revendications 1 à 13, **caractérisé en ce que** la barrière d'adhérence (4) présente un renforcement (18) pour l'étirage de la barrière d'adhérence (4) dans l'état détendu, dans lequel la barrière d'adhérence étirée (4) s'étend essentiellement parallèlement à la face de base (2).

15. Implant selon l'une quelconque des revendications 1 à 14, **caractérisé en ce que** la barrière d'adhérence (4) présente un renfort de protection flexible local (19) pour la fixation à la face de base (2) et/ou à la région antérieure (10), renfort de protection (19) qui est de préférence intégré dans un matériau de la barrière d'adhérence (4).
